# EUROPEAN PATENT APPLICATION

(11) **EP 1 449 533 A1**
(43) Date of publication of application: **25.08.2004**
(21) Application number: 03003281.7
(22) Date of filing: 24.02.2003
(51) Int. Cl.: A61K 31/79, A61K 9/127

(54) **Use of PVP-iodine liposomes for the treatment of acne**

(71) Applicant: EURO-CELTIQUE S.A., 2330 Luxembourg (LU)
(72) Inventor: Reimer, Karen, 65582 Hambach (DE); Fleischer, Wolfgang, 55218 Ingelheim (DE); Hopp, Michael, 65428 Russelsheim (DE)
(74) Representative: Maiwald Patentanwalts GmbH

(57) **Abstract**

The invention concerns a method for the production of a pharmaceutical preparation for the treatment of acne forms that is characterized in, that the preparation comprises at least one antiseptic compound associated with a particular carrier.

## Description

The present invention concerns the use of PVP-iodine liposomes for treatment of acne. The present invention also concerns methods for producing a pharmaceutical preparation for the treatment of acne wherein the preparation comprises at least one antiseptic compound in a pharmaceutically effective amount combined with a particular pharmaceutically acceptable carrier.

Acne is a skin disease of different forms and causes. Formation for pustules, abscesses and scars due to stuffed hair follicles (comedones) can be some of the features of acne. The most common form of acne which occurs normally during puberty and may last until the age of 30 is *Acne vulgaris.* This acne form is characterized by the occlusion of follicles and subsequent formation of comedones due to sebaceous gland hyperplasia and a follicular hyperkeratosis in the sebaceous gland-rich skin parts such as the face, the neck, the breast and the back. Skin lesions are another typical symptom *of Acne vulgaris.* An interplay of genetic dispositions, seborrhoea, certain bacteria such as *Propionibacterium Acnes, Propionibacterium granulosum* and *Propionibacterium avidum,* hormonal influences and immune reactions due to inflammatory stimuli are often conditions for the occurrence *of Acne vulgaris.*

*Acne vulgaris* can occur in forms of different severity such as *Acne comedonica, Acne papulopustulosa* and *Acne conglobata.* Whith the occurrence of open and closed comodones being typical for *Acne comedonica, Acne papulopustulosa* is characterized by the transition to inflammatory pustules and papules. *Acne conglobata,* which is the most severe form *of Acne vulgaris,* is characterized by large inflammatory knots, abscesses, fistula, deep scars and celoides in the above-mentioned skin parts, but also at the extremities and at the back side.

There are other known acne forms besides *Acne vulgaris. Acne venenata* (also called contact acne) is caused by halogenated hydrocarbons, lubricating oils, tar and pitch, but may also occur as a side effect due to the intake of pharmaceuticals such as corticosteroids, androgens, thiamine and cobalamin. Another contact acne is *Acne cosmetica* that occurs mainly with women after prolonged application of over-fatty cosmetics. Other acne forms comprise *Acne aestivalis, Acne excoriée des jeunes filles, Acne fulminans* and *Acne tetrade.*

Independent of the particular acne form, affected persons usually experience these skin diseases as extremely unpleasant and troubling. Particularly teenagers experience a serious loss of self-esteem and of their perception by others due to the skin inflammations in their face. Mainly due to this severe suffering of both teenagers and also patients who are affected by other forms of acne than the pubertal *Acne vulgaris,* there is a strong need for pharmaceutical preparations that allow for a efficient treatment of both the mild and severe forms *of Acne vulgaris* as well as of other acne forms. Such preparations should particularly bring about the scar-free healing of the comedones, the pustules, the abscesses and the pus filled pimples.

From the prior art a multitude of pharmaceutical preparations for the treatment of different acne forms is known. In those keratolytic, anti-microbial and anti-seborrhoic compounds are mainly used for therapeutic treatment.

Antibiotics are mainly used as anti-microbial compounds as they should prevent the bacterial colonisation of comedones that usually develops upon the often already existing seborrhea. The killing of bacteria is particularly important as they generally initiate skin inflammations due to their production of free fatty acids and tissue-harming enzymes. In the past antibiotics have been selected mainly due to their ability to kill gram-positive organisms such as *Corynebacterium acnes, Propionibacterium acnes, Propionibacterium granulosum* and *Propionibacterium avidum* as well as *Staphylococcus epidermis* and *Pityrosporon* (Millikan et al. 1976, Cutaneous medicine for the Practitioner, 17, 394-398). A general drawback of the use of antibiotics is that infectious bacteria may show primary resistances against these compounds or may develop secondary resistances upon contact.

Additionally antibiotics very often lead to a sensibilisation of the patient. Antibiotics and compounds such as erythromycin, clindamycin and acelaic acid have therefore been mainly topically applied during the treatment of acne. The topic application of antibiotics-comprising preparations for the treatment of acne can induce a reduction of skin surface infections with *Staphylococci;* it does, however, not allow for the effective removal of bacteria such as *Corynebacterium acnes* or *Pityrosporon* that occupy the anaerobic part of the follicles. Prior art pharmaceuticals preparations for the treatment of acne that comprise antibiotics as essential compounds and that are to be applied topically, have the drawback, that severe forms of acne can not be cured, most probably because the compounds do not penetrate deep enough into the hair follicles.

Systemic application of antibiotics for the treatment of acne is to be avoided per se because of the already afore-mentioned danger of developing resistances. Moreover, due to the local occurrence of acne and physiological stress generally connected with the intake of antibiotics, systemic application does generally not seem appropriate. The systemic application of antibiotics such as tetracylcline for the treatment of acne is therefore indicated only in severe cases.

As bacterial infections of hair follicles are only one of the inflammation-inducing causes of acne besides hyperkeratosis, prior art pharmaceutical preparations that comprise antibiotics for the topical treatment of acne, have often been combined with additional keratolytic compounds such as benzoyl peroxide or isotretinoin. As with the topically applied antibiotics, these keratolytic compounds have the general drawback that, due to their poor penetrance into the hair follicle, they can only be used for treatment of mild forms *of Acne vulgaris* such as *Acne comedonica,* but not for the treatment of severe forms such as *Acne papulopustulosa* or *Acne conglobata.*

Other compounds that are known in the art and that may be used for the treatment of mild forms of acne are acelaic acid or hexachlorophene. These compounds show a strong anti-microbial efficacy; they cause, however, serious side effects such as skin irritations. Hexachlorophene has therefore not been used anymore for treatment of acne.

For treatment of another cause of acne, namely hyperseborrhea, anti-seborrhoic compounds such as anti-androgens (e.g. cyproterone acetate) or oestrogens (e.g. mestranol) are administered. It is obvious that the systemic application of these compounds for the treatment of acne goes along with serious side effects for the patient due to their influence on hormone metabolism. Correspondingly, the use of these compounds is only indicated for the treatment of very severe forms of acne.

And additional compound that has been used for the treatment of acne in the past is PVP-iodine. The use of antiseptics and particularly halogen-releasing antiseptics such as povidone-iodine, also known as polyvidone-iodine or PVP-iodine, e.g. poly(1-vinyl-pyrrolidine-2-on)-iodine complex, has the general advantage that the development of resistances is avoided while retaining at the same time a high anti-microbial efficacy. Moreover, these antiseptic compounds rarely dispose allergenic properties compared to antibiotics.

In the prior art PVP-iodine has been used for the treatment of acne always in the form of a soap solution, that is commercially available under the designation Betadine (in the US) or Betaisadona (in Europe). Studies concerning the efficacy of this PVP-iodine complexes containing soap solution showed consistently that PVP-iodine in the form of the soap solution can be used for the topic treatment of mild forms *of Acne vulgaris* (Brown et al. 1977, Brit. J. Clin. Practice, 31, 218-219; Khan et al., 1979, Brit. J. Clin. Practice, 33, 289-290; Millikan et al., 1976, vide supra).

It emerges unambiguously from these studies that the used PVP-iodine soap solutions do not provide for a high efficiency for the treatment of acne as in almost all cases patients were treated also with antibiotics. Even in these cases up to 15% of the patients showed no improvement upon the simultaneous topic application of PVP-iodine soap solutions and antibiotics (Brown et al., 1977, vide supra). This shows that a significant portion of patients could not be helped by application of PVP-iodine soap solutions even if mild forms of acne were treated and treatment was supported by a therapy with antibiotics and hormones.

In cases where patients received no supporting systemic or topic treatment with antibiotics, sulfonamides or keratolytic compounds such as benzoyl peroxide, the proportion of patients where the topic application of the PVP-iodine soap solution would not allow for an efficient treatment of mild forms *of Acne vulgaris* increased even up to 33% (Khan et al., 1979, vide supra). In case patients with more severe forms of acne were treated with the PVP-iodine soap solution and a supporting systemic tetracycline therapy were treated, it became clear that despite the systemic application of tetracycline and the simultaneous topic application of the PVP-iodine soap solution no improvement of acne would occur (Millikan et al., 1976, vide supra).

The afore-mentioned soap solutions which are usually of a brown colour have the additional drawback that the treated skin may show coloured patches. Even though the colour may be removed by thorough washing, this is not acceptable where sensitive and already predamaged skin areas are to be treated.

This shows that the afore-mentioned compounds allow for a slight improvement of mild forms *of Acne vulgaris* upon topic application at best. Even in these cases the use of keratolytic compounds such as benzoyl peroxide, tretinoin or salicylic acid can lead to typical irritative side effects particularly upon treatment of sensitive skin and frequent administration (Haut, number 5, September 2000). If more severe forms of acne are to be treated, the afore-mentioned compounds have to be applied systemically (with the exception of PVP-iodine). Even then an improvement of severe forms of acne is not guaranteed, (see e.g. for systemic application of tetracycline) and the side effects (e.g. due to hormonal treatment) are significant for patients so that a systemic application of the afore-mentioned compounds seems generally not appropriate.

The fact, that by topic application of the afore-mentioned pharmaceutical preparations only mild forms of acne can be treated and even then often only in an insufficient manner, has led to the assumption that the respective compounds do not penetrate deep enough into the hair follicles. Accordingly, suggestions have been made concerning the formulation of pharmaceutical preparations treating acne, that may allow for a deep penetration of the compounds into the skin tissue and the hair follicle.

The use of liposomes for producing pharmaceutical preparations that allow for the treatment of acne is known in the art. Lactic acid, benzoyl peroxide, acelaic acid and retinoides such as tretinoin, isotretinoin, adapalen and tazaroten have been suggested as compounds that may associate with liposomes (Rippke, 2000, Haut, Number 5; Patel et al., 2001, Drug Dev. Ind. Pharm., 27, 863-869; Gollnick et al., 1998, Dermatology, 196, 119-125; Patel et al., 2000, Pharm. Dev. Technol., 5, 455-464). A drawback of these liposomal preparations is that due to the better penetration of the preparation into the skin a higher absorption of the compounds occurred which led to disadvantageous reactions for the patients (Gollnick et al., 1998, vide supra). Moreover, these liposomal preparations are only suitable for treatment of mild forms *of Acne vulgaris* but not for the treatment of the more severe forms that are characterized through a significant scar formation of the tissue.

In none of the afore-mentioned prior art documents are hints towards the production of liposomes that contain PVP-iodine for the treatment of acne.

The use of antiseptics and/or bound-healing promoting agents for external application to humans and animals is disclosed in EP 0 639 373. Specifically, liposome preparations of PVP-iodine are shown therein to be topically applicable to the external parts of the eye mainly for purposes of complete disinfection. These preparations generally take the form of a cream, an ointment, a lotion, a gel or a drop formulation.

Liposomes are well-known drug or compound carriers and correspondingly the application of medicaments in liposomal form has been subject of investigation for quite some time. An overview concerning the administration of compounds in liposomal forms to the skin is provided by the review "Targeted delivery to the pilosebaceous unit via liposomes" (Lauer, A.C. et al., 1996, Advanced Drug Delivery Reviews, 18, 311-324). The physical-chemical characterisation of liposomal preparations and their therapeutic application for the treatment of the pilosebaceous unit are described therein. Compounds that have been investigated for delivery via liposomes include e.g. anti-cancer agents, peptides, enzymes, anti-asthmatic and anti-allergic compounds and, as mentioned above, also antibiotics.

Liposomes or other particles are not known as carriers of antiseptic compounds for the treatment of acne.

One objective of the present invention is to provide for a well-tolerated, easy applicable pharmaceutical preparation for the topical treatment of acne, that allows for a lasting, efficient and scar-free treatment also of severe acne forms. It is also an objective of the present invention to provide a method for producing a pharmaceutical preparation for application of at least one antiseptic compound for the treatment of all forms of acne wherein the preparation comprises at least one antiseptic compound combined with a particular, pharmaceutically acceptable carrier. These and other objectives of the present invention, that will become obvious from the description, are solved by the subject-matter of the independent claim. Preferred embodiments are defined by the dependent claims.

It has been surprisingly found that pharmaceutical preparations according to the invention that comprise a antiseptic compound, as e.g. PVP-iodine, in a pharmaceutically effective amount combined with particles, as e.g. liposomes, can be ideally used to treat efficiently both the different forms *of Acne vulgaris* and other acne forms by topic application. The novel use according to the invention of the particles-containing preparations that comprise antiseptic compounds, as e.g. PVP-iodine, for the treatment of acne moreover shows the surprising advantage that a scar-free healing of the skin damages caused by acne is possible.

The invention is thus premised on the surprising fact, that particulate carriers, especially liposomes, are exceptionally well suited as carriers for antiseptic agents, especially for PVP-iodine, for the application and treatment of mild and severe forms *of Acne vulgaris* and others of the afore-mentioned acne forms. The preparations according to this invention permit protracted release of the compound(s) and provide an extended and topical activity at the desired locus of action or interaction with the respective skin cell surfaces. Without wanting to by bound to a specific scientific theory, it is assumed that the outstanding effect of the PVP-iodine liposomes according to the invention is due to the deeper penetration of the liposomes into the damaged skin areas compared to conventional preparations. In this way, the compound(s) is/are transported in more efficiently way to the damaged skin parts. However, it is then surprising that such a radically effective substance class such as antiseptics does not impinge on the healing process of the especially sensitive and damaged tissue and can even suppress the formation of scar tissue, neoplasms, intergrowth etc.. This may be due to the granulation and epithel-forming effect of the liposomal preparations.

In the context of the present invention the term "acne" comprises inflammatory skin damages that are caused by a combination of bacterial infection, hyperkeratosis of the hair follicle and a hypersebarrhoea. The term "acne" particularly comprises the different forms *of Acne vulgaris* such as *Acne comedonica,* but also more severe and most severe forms such as *Acne papulopustulosa* or *Acne conglobata.* In the context of the present invention other acne forms comprise the afore-mentioned *Acne aestivalis, cosmetica, excoriée des jeunes filles, fulminan, neonatorum, venenata* and *tetrade.*

In the context of the present invention, antiseptic compounds first and foremost comprise compounds designated as antiseptics and used as such in the prior art. According to the invention these compounds especially comprise such disinfecting compounds that are pharmaceutically acceptable and can be used for the treatment of the various forms of atopic dermatitis mentioned above by topical application, as long as they have a formulation according to the invention. The antiseptic compounds preferably comprise, among other things, oxygen-releasing or elementary halogene-releasing compounds as well as metal compounds, such as silver and mercury compounds. More preferably, the antiseptic compounds according to the invention comprise halogene-releasing compounds such as iodine, iodine complexes and PVP-iodine which contain the iodine in elementary form.

In the context of the present invention the term "pharmaceutically effective amount" refers to an amount of the antiseptic(s) in the preparations that is sufficient for treating the various afore-mentioned acne forms efficiently.

According to the invention the inventive preparations can be used to treat the different forms of acne upon topic application so that the inflamed hair follicles, comedones, pustules, papules, inflammatory knots, abscesses, fistula and celoides which have developed as a consequence of the acne regress and heal in effect completely, i.e. scar-free.

This effect is due to the surprising and unexpected fact, that hyperkeratosis and uncontrolled proliferation of tissue can be avoided by use of inventive preparations such as PVP-iodine-containing liposomes. Thereby, the serious functionally and cosmetically relevant skin damages, that are one threatening consequence of the uncontrolled proliferation during the formation of new skin tissue, can be prevented. Cosmetically, the scar-free healing of acne is particularly important as the typically face-attacking acne usually is sensed as extremely unpleasant by the affected persons. The fact, that use of e.g. PVP-iodine-containing liposomes allows for such an efficient treatment of the various acne forms was especially surprising as it could not be expected that by use of preparations that comprise only one active compound the various reasons underlying acne (such as hyperseborrhea, hyperkeratosis as well as bacterial and viral infections and inflammations) can be simultaneously and efficiently treated by topic application without the occurrence or remains of scar tissue. Application of PVP-iodine liposomes according to the invention ensures moreover that the damaged skin parts remain scar-free. Additionally PVP-iodine liposomes provide for sufficient moisture to ensure an effective healing process.

PVP-iodine liposomes according to the present invention may therefore also be used for cosmetic purposes.

The formation of scar tissue on the skin can be reduced and hyperkeratosis may be completely repressed through the use of preparations according to the invention. Intergrowth or the formation of neoplasm that may lead to scars are significantly reduced upon use of e.g. PVP-iodine-containing liposomes for the treatment of different acne forms. Moreover, in the course of the acne treatment the use of the afore-mentioned preparations effects also an efficient killing of bacterial infections that frequently make up a major component of the acne. Thus, antibiotics do not have to be administered as a supplementary therapy and the danger of resistance development does accordingly also not exist.

The composition of the liposomes, the concentration of the active compound(s) and methods for producing the PVP-iodine liposomes or preparations comprising an antiseptic compound in a pharmaceutically effective amount combined with the particulate carriers are presented below. If, for purposes of demonstration, liposomes and PVP-iodine are mentioned, the person skilled in the art is well aware that other carriers and other antiseptics may be formulated in analogous manner and may thus also be used for the same purposes.

The preparations according to the invention may also be used for treating the symptoms of the various forms of acne such that a cosmetically acceptable and satisfying result is achieved for the affected persons. This aspect of the invention may also be designated as cosmetic remodelling.

Preparations according to the invention whose use permits the efficient treatment of the various forms of acne can be produced by loading liposomes with PVP-iodine according to methods known in the art. The nature or composition of the liposomes is generally not decisive for the treatment success and can vary. The liposomal preparation, as for example described in EP 0 639 373, can be administered in different forms including, e.g. an ointment, a cream, a spray, a lotion, a solution, a suspension, a dispersion or a gel. The disclosure of EP 0 639 373 is incorporated herein by reference.

Preferably, the liposome-forming material is selected so that it does not react substantially or hardly reacts at all with the antiseptic compounds. One will therefore try to keep the content of chemically reactive substances as low as possible, if the antiseptic might be a reaction partner. If the antiseptic can release oxygen or halogen atoms, as is the case with PVP-iodine, one will use e.g. cholesterol which has a reactive double bond only in small amounts, if at all. In any case, one will choose the amount of such potentially reactive liposome-forming substances such that the pharmaceutical preparations have a sufficient storage stability of at least one, or even of at least two years (in compliance with statutory regulations). The storage conditions may comprise a temperature range of approximately -20 °C to approximately 60 °C.

The preparations according to this invention often contain the active compound(s), such as PVP-iodine, encapsulated in the particulate carrier, especially in liposomes. However, it may also occur that there is an amount of compound not contained inside the carrier. The compound(s) may also be associated with the surface of the particle carriers as e.g. liposomes.

In one embodiment of the invention the major part or even the whole amount of the active compund(s) may be located outside the particulate carriers as e.g. liposomes.

The preparations according to the invention then may show a marked initial effect which is observed in addition to the slower, protracted release of the active agent from the carrier. This effect is especially observed where the carrier comprises liposomes. Without wishing to be bound to any theoretical explanation, it is presently assumed that in addition to the active agent encapsulated inside the liposomes, some active agent is present outside of the liposomes, and probably loosely bound to the outer surfaces of the liposomes. This could be due to complex association of active compound molecules with the liposomal membrane, or it could be due to active compound molecules forming a layer on the liposomal surface, which layer partly or even fully coats the liposome externally. The type and amount of this initial compound effect can e. g. be influenced by choice of the concentration parameters.

In the context of the present invention, protracted or prolonged release means that the active compound(s) is/are released form the pharmaceutical preparation over a time period between 1 to 24 hours.

The association of antiseptic compounds with liposomes, i.e. that active compounds can be included in the interior of liposomes or, depending on the circumstances, can also associate with the surfaces, depends among other things on the components used for formation of the liposomes.

In a preferred embodiment, preparations according to the invention used for the treatment of the various forms of acne can comprise, besides the antiseptic compound also other anti-inflammatory agents and agents promoting wound-healing.

These additional anti-inflammatory comprise e.g. phenolic compounds, detergents, alcohols, organic disinfectants including among other things formaldehye-releasing compounds, phenolic compounds including alkyl and aryl phenolic compounds, as well as halogenated phenolic compounds, chinolines, acridines, hexahydropyrimidines, quaternary ammonia compounds, iminium salts and guadinines. Agents promoting wound-healing comprise those substances that have been described in the literature for such applications. Such compounds comprise substances that are known for promoting the formation of epithelial tissue. These include vitamins, particularly from the vitamin B group, alantoin, some azulenes, etc.

In some embodiments of the present invention, preparations according to the present invention comprise antiseptic compounds, preferably PVP-iodine, and may also comprise compounds such as agents promoting wound-healing or anti-inflammatory compounds.

Inventive preparations can also contain other customary agents, including adjuvants and additives, antioxidants, conserving agents or consistency-forming agents such as viscosity-adjusting additives, emulgators, etc. The person skilled in the art will select these adjuvants and additives in such a way that the ability of preparations, substantially consisting of particulate carriers such as liposomes and antiseptic compounds such as PVP-iodine, to efficiently treat the various forms of acne, is not impaired. Additives may also comprise salts that allow for the regeneration of the active compound, such as the released halogen atom in case of halogen-releasing compounds. In the case of PVP-iodine such an additive may be KIO₃. Other additives that mediate or enhance penetration of the liposomes into the skin may also be part of the inventive preparations. Such additives comprise e.g. DMSO.

The amphiphilic substances generally known in prior art to form liposome membranes can be employed in the context of the invention as long as they are pharmaceutically acceptable for the intended application. Presently, liposome-forming systems comprising lecithin are preferred. Such systems can comprise hydrogenated soy bean lecithin besides cholesterol (if suitable despite its reactivity) and disodium succinatehexahydrate. Preferably one will make sure that the liposome-forming materials do not show any reactivity with the antiseptics in order to ensure the required storage stability of commercial products. Due to its double-bond reactivity high cholesterol contents will be avoided where it is to be formulated in connection with oxygen-releasing or halogen-releasing antiseptics (such as PVP-iodine). It is presently specifically preferred to use hydrogenated soy bean lecithin as the sole membrane-forming agent. Commercially available products such as Phospholipon® 90 H (Aventis, Germany) or Lipoid S 100-3 (Lipoid GmbH, Germany) are also preferred.

As can be taken from the review of Lauer A.C. et al. 1995 (*vide supra*) phospholipid-based liposomes may also be generally used for production of liposomes that discharge their cargo into the skin. According to this review, the use of non-ionic liposomes, which can be formed with phosphatidylcholin, is also an option. The presence of sebum in the hair follicle may be relevant for the choice of components that the liposomes are formed from. Other components that may be used for the formation of micelles are also known to the person skilled in the art and may be used for the production of preparations according to the invention.

The known prior art methods for forming liposome structures can generally be used in the context of the invention. Broadly, these methods comprise mechanical agitation of a suitable mixture containing the membrane-forming substance and water or an aqueous solution. Filtration through suitable membranes is preferred in order to form a substantially uniform liposome size.

The average size of the liposomes according to this invention can vary over a broad range, generally from about 1 nm to about 100 µm. Liposomes or particulate carriers having diameters in the range of about 1 µm and 70 µm are preferred. The person skilled in the art knows that the efficiency of liposomal penetration into the skin increases with decreasing diameter and that therefore liposomes having diameters of about 0,01 µm to 10 µm, of about 0,01 µm to 1 µm, but also of about 5 to 7 µm or about 5 µm may be used. Generally the size of liposomes should be selected such that a good penetration into the skin is guaranteed. Particularly preferred embodiments of the invention therefore comprise liposomes having a diameter of between about 0,01 µm to 25 µm and preferably around 1 µm.

Liposomes in more fluid preparations may be generally more suited for treatment of bacterial infections, while liposomes in more gel-like formulations are generally better suited for treatment of viral infections. It seems that symptoms that are due to viral infections are preferably treated with preparations according to the invention that allow for longer contact times with the affected body areas. Symptoms due to bacterial infections may be treated preferably with preparations that have rather short contact times with the affected body areas.

Thus it is preferred that inventive preparations for the treatment of the various forms of acne comprise liposomes in a more fluid preparation such as a solution, dispersion, suspension, lotion or a gel of rather low viscosity. Moreover, these preparations preferably comprise liposomes of rather small size such as liposomes having a diameter between about 1 µm and 30 µm, preferably between about 1 µm and 20 µm, more preferably between 1 µm and 10 µm and most preferably at around 5 µm.

Generally, liposomes having a rather small average diameter are better suited for production of solutions, dispersions, suspensions. Such rather small diameters typically comprise diameters of around 1 µm to 10 µm, or even smaller in the case of solutions. In contrast, gel or ointment formulations may comprise liposome of a size of about 1 µm to 50 µm.

Where alternative particulate carriers are used, they are generally prepared as known in the art. Thus, microspheres which are used to deliver a very wide range of therapeutic or cosmetic agents, are made as described for example in WO 95/15118.

Nanoparticles may in some cases be used, provided that they can be loaded with a sufficient amount of active agent and can be administered to the lower respiratory tract according to this invention. They can be prepared according to the methods known in the art, as e. g. described by Heyder (GSF München) in"Drugs delivered to the lung, Abstracts IV, Hilton Head Island Conference, May 1998.

Methods using a pulse laser deposition (PLD) apparatus and a polymeric target to apply coatings to drug powders in a short non-aqueous process are also suitable for the formation of particulate preparations according to this invention. These have e. g. been described by Talton et al., "Novel Coating Method for Improved Dry Delivery", Univ. of Florida UF 1887 (1998).

A further suitable delivery system employs Large Porous Particles as disclosed by David A. Edwards et al. in "Large Porous Particles for Pulmonary Drug Delivery" (Science, 20. June 1997, Vol. 276, p 1868-1871).

Generally, the concentrations in the preparation, particle sizes, active agent loadings etc. will be selected for such alternative carriers to correspond basically to the parameters discussed herein with respect to liposome preparations. Selecting and providing such parameters based inter alia on straightforward experimentation, is well within the skill of an ordinary worker experienced in this art.

According to the invention, use of inventive liposomal preparations is with the treatment of the various forms and severity grades of acne, particularly if inventive preparations comprise PVP-iodine and liposomes. The use of inventive liposomal preparations for treatment of various forms of acne, irrespective of the severity grade, has the advantage that, since no antibiotics are used, no resistances develop thus leading to a reduction of allergic reactions.

Moreover, as no keratolytic compounds such as benzoyl peroxide or hormones are used, typical contact and skin irritations do not occur. Generally, the use of inventive preparations for topical treatment of various forms of acne allows for efficient treatment of the various forms of acne with significantly reduced side effects and without formation of scar tissue. A systemic application of the compounds with antibiotics or hormones, as is indicated for treatment of severe acne forms, is not necessary. Undesirable side-effects are therefore generally avoided.

Another advantage of the inventive liposomal antiseptic preparations is that they are also active against viruses, which antibiotics are not. Thus, inflammatory reactions which may also contribute to the acne phenotype, but which are not caused by bacterial infections, can also be efficiently treated in the course of acne treatment with the inventive liposomal preparations. Moreover, liposomal preparations with one antiseptic compound, such as PVP-iodine, allow for protracted compound-release from the liposomes. This leads to a prolonged efficacy of the anti-microbial substance and thereby allows for less frequent application of the preparation compared to known antiseptic solutions.

Preparations according to this invention can take a variety of forms, including pharmaceutically acceptable solid or liquid formulations such as emulsions, dispersions, suspensions, solutions, gels, ointments, waxes, spray, lotions, etc. The formulation as a Hydrogel is preferred. If in the context use is made of the term "gel", this thus always includes the preferred embodiment of a Hydrogel.

Generally, the amount of active agents in an inventive preparation will be determined by the desired effect on the one hand and the carrying capacity of the carrier preparation for the agent on the other hand. Broadly speaking, the amount of active agent in an inventive carrier preparation can range in concentrations between the lower limit of effectiveness of the agent and the maximum loading of the agent in the respective carrier preparation. It is understood that the active compounds are present in the inventive preparations in a pharmaceutically sufficient amount, i.e. in an amount sufficient for treatment of the different forms of acne.

More specifically, for an antiseptic agent, such as povidone iodine, a solution, dispersion, oil , ointment or gel in an inventive carrier preparation, especially where the carrier is a liposome preparation, can contain between 0.1 and 10 g of agent in 100 g of preparation. Such a preparation will then typically contain between 1 and 5 g of liposome membrane-forming substance, especially lecithin, per 100 g of preparation.

Usually preferred active compound concentrations such as of e.g. PVP-iodine concentration are normally between 1% to 10% and preferably 1 % to 5% by weight.

In a lotion, which can be a hydrophilic or a lipophilic lotion, a typical range of active compound such as PVP-iodine will be between 0.5 and 10 g compound, and between 1 and 5 g, preferrably about 4 g of liposome membrane forming agent such as hydrogenated soy bean lecithin per 100 g of lotion. In the case of a hydrophilic lotion, electrolyte solution will often be used in preparing the liposome-containing lotion.

A lipophilic lotion will often be made from compound, membrane-forming substance and lipophilic formation agents such as medium chain length triglycerides, etc.

A hydrophilic cream comprising an inventive liposome preparation will generally comprise between 0.1 and 10 g agent, such as PVP-iodine, together with between about 1 and 10 g membrane forming substance and further typical O/W cream forming additives per 100 g of cream.

A comparable amphiphilic cream according to the invention will have similar contents of agent and membrane forming substance such as lecithin, and will have the typical further additives of an amphiphilic cream.

A hydrophilic ointment according to the invention can broadly comprise between 0.1 and 10 g compound and between 1 and 10 g liposome membrane forming substance such as lecithin, together with typical prior art ointment basis substances such as Macrogol (TM) and water in 100 g of ointment.

A non-alcoholic hydrogel according to the invention could broadly comprise between 1 and 5 g compound such as PVP-iodine, approximately 2-4 g lecithin and gel-forming substances such as Carbopol (TM), with pH-adjusting agent and water to form 100 g of hydrogel.

An inventive aerosol or spray preparation will often comprise up to 50 mg, but could comprise up to and above 100 mg of liposomal active compound formulation per unit spray dose. The spray preparation will typically comprise at least 10 % wt of compound agent such as PVP-iodine in the loaded liposomes (or alternative carrier particles), but may comprise up to 50 % wt or even more of active agent. Where the active agent is PVP-iodine, the amount of available iodine will generally be about 10 % wt (based on PVP-iodine).

More specific formulations are notable from the embodiment examples.

The features and advantages of this invention will become clear in more detail from the ensuing description of preferred embodiments. In these embodiments, which include a best mode, povidone iodine is exemplified as an antiseptic agent and liposomes are chosen as the carrier. This should however not be construed as a restriction of this invention to antiseptic agents alone or, among antiseptic agents, to povidone iodine, and/or to liposomes as the carrier, although such preparations are specifically preferred. According to the invention other particulate carriers such as "large porous particles" or other micelles, nanoparticles, etc. can be formulated with PVP-iodine in order to produce preparations that allow for an efficient treatment of the various forms of acne. Correspondingly, other halogen-releasing antiseptics can be formulated with liposomes into a preparation that also allows for the efficient topical treatment of acne. One preferred method for producing the invention's liposomes can generally be described as follows:

The lipid membrane-forming components, e. g. lecithin, are dissolved in a suitable solvent such as chloroform or a 2: 1 mixture of methanol and chloroform and are filtered under sterile conditions. A lipid film is then produced on a sterile high surface substrate, such as glass beads, by controlled evaporation of the solvent. In some cases, it can be quite sufficient to form the film on the inner surface of the vessel used in evaporating the solvent without using a specific substrate to increase the surface.

An aqueous system is prepared from electrolyte components and the (one or more) active agents to be incorporated in the liposome preparation. Such an aqueous system can e. g. comprise 10 mmol/l sodium hydrogen phosphate and 0.9 % sodium chloride, at pH 7.4; the aqueous system will further comprise at least the desired amount of the active agent, which in the embodiment examples is povidone iodin. Often, the aqueous system will comprise an excess amount of agent or agents. The pH of the buffer solutions may be varied.

The following table discloses buffer solutions which may be used for preparation of liposomal dispersions.

| | pH 5 | pH 5 | pH 5 | pH 6 | pH 7.4 | pH 7.4 |
|---|---|---|---|---|---|---|
| Na₂HPO₄ | 0.0114 g | | 0.1436 g | | | |
| Na₂HPO₄x2H₂<O | | 1.8334 g | | 2.2464 g | 0.445 g | 0.89 g |
| KH₂PO₄ | 0.8970 g | | 0.7973 g | | | |
| Citric acid | | 0.9312 g | | 0.7085 g | | |
| 1 M HCl | | | | | pH adjust. | pH adjust. |
| Water, purified | ad 100 ml | ad 100 ml | ad 100 ml | ad 100 ml | ad 100 ml | ad 100 ml |

Preparations may then finally be adjusted with NaCl or Glycerol to an isotonic range (250-350 mOsmol/kg). Depending on the PVP-iodine concentration the salt strength may also be varied (as e.g. shown for buffer solutions with pH 7.4 in the above table).

The liposomes are generally formed by agitating said aqueous system in the presence of said film formed by the lipid components. At this stage, further additives can be added to improve liposome formation; e. g. sodium cholate. Liposome formation can also be influenced by mechanical action such as pressure filtration through e. g. polycarbonate membranes, or centrifuging. Generally, the raw liposome dispersion will be washed, e. g. with electrolyte solution as used in preparing the above-described solution of the active agent.

When liposomes with the required size distribution have been obtained and washed, they can be redispersed in an electrolyte solution as already described, often also comprising sugars such as saccharose or a suitable sugar substitute. The dispersion can be freeze-dried, and it can be lyophilysed. It can, prior to use, be reconstituted by addition of water and suitable mechanical agitation at the transition temperature of the lipid component, which for hydrogenated soy bean lecithin is e. g. 55 C.

In the following Examples, hydrogenated soy bean lecithin (EPIKURON (TM) 200 SH obtainable from Lukas Meyer, Germany or PHOSPOLIPON (TM) 90 H obtainable from Nattermann Phospholipid GmbH, Germany) was used. However, other pharmaceutically acceptable liposome membrane forming substances can be used instead, and the person skilled in the art will find it easy to select suitable alternative liposome forming systems from what is described in prior art.

The person skilled in the art is well aware that the liposomes-containing dispersion may comprise additional additives and adjuvants that can influence the appearance of the liposomal preparations. The inventive liposomal dispersion may contain e.g. colour pigments that ensure that the slightly yellow or brown colours that are due to PVP-iodine or released iodine are not visible. In the same manner, liposomes or the pharmaceutical liposome-containing preparations may comprise additives that influence the consistency and the smell of the preparations.

The person skilled in the art is well aware that the choice of these additives and adjuvants depends on the intended application form of the preparation (e.g. as ointment, gel or solution) and may be influenced by aesthetic considerations (such as colour and smell).

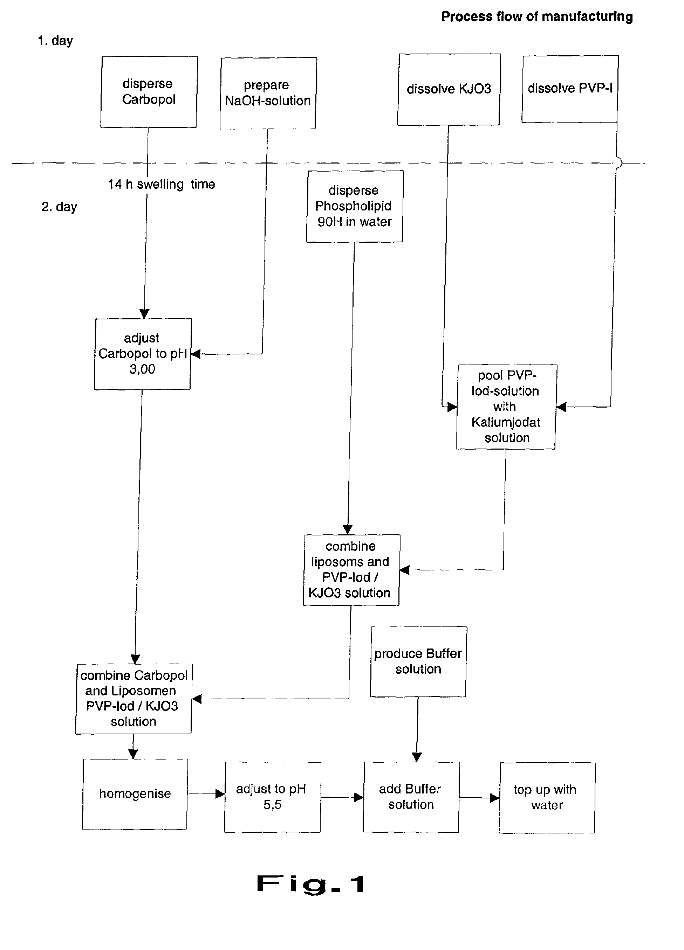

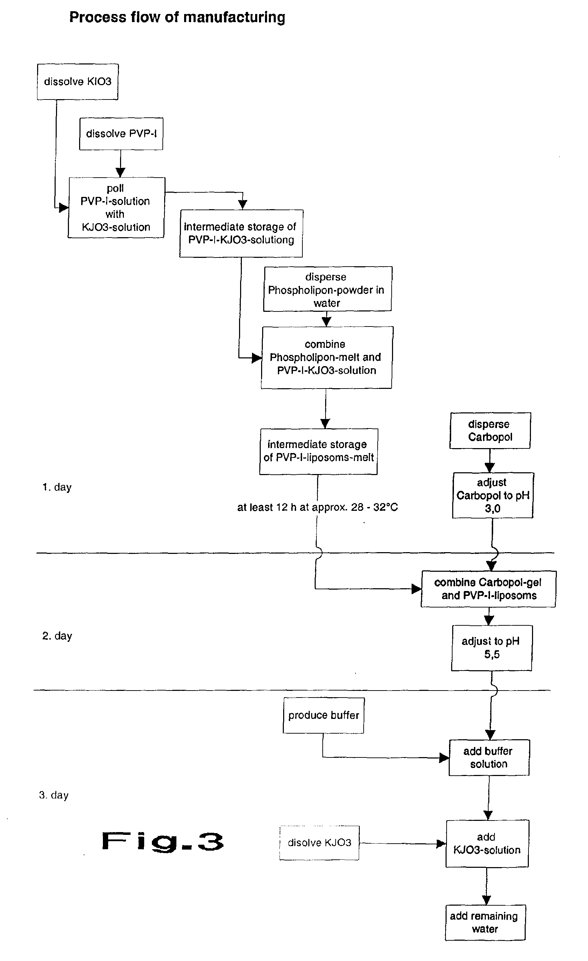

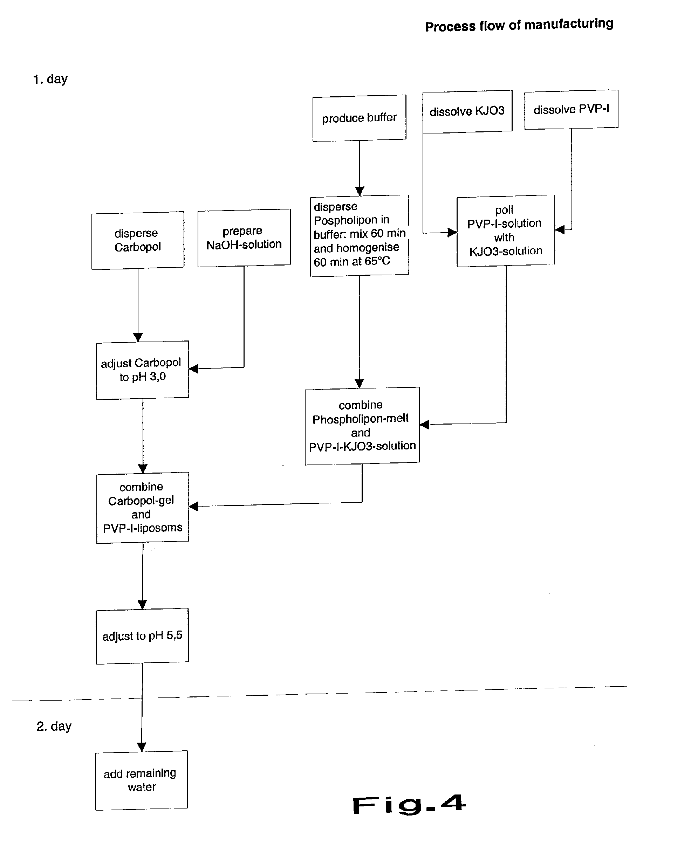

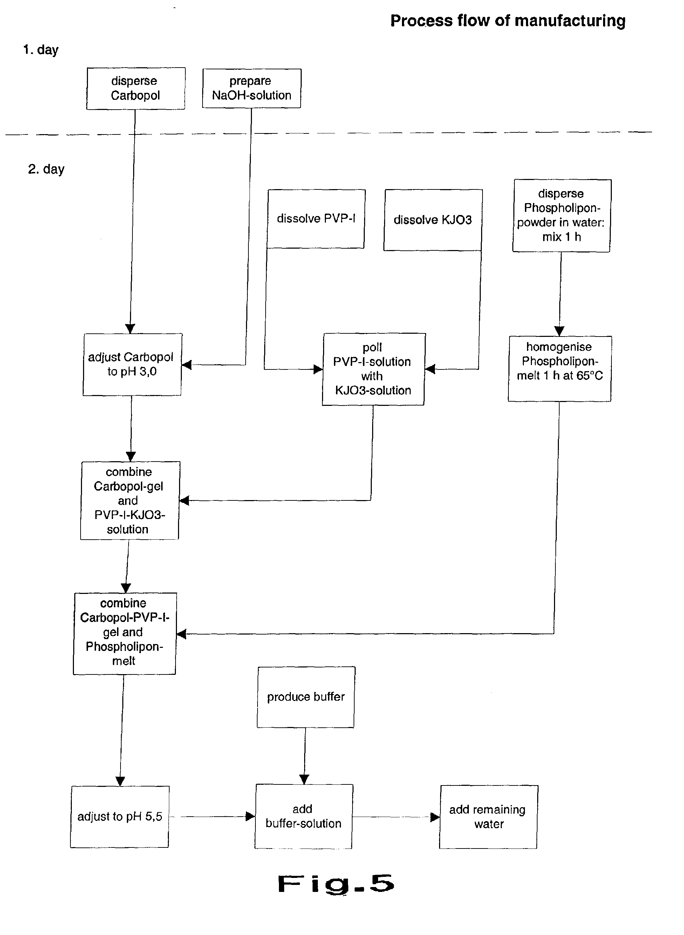

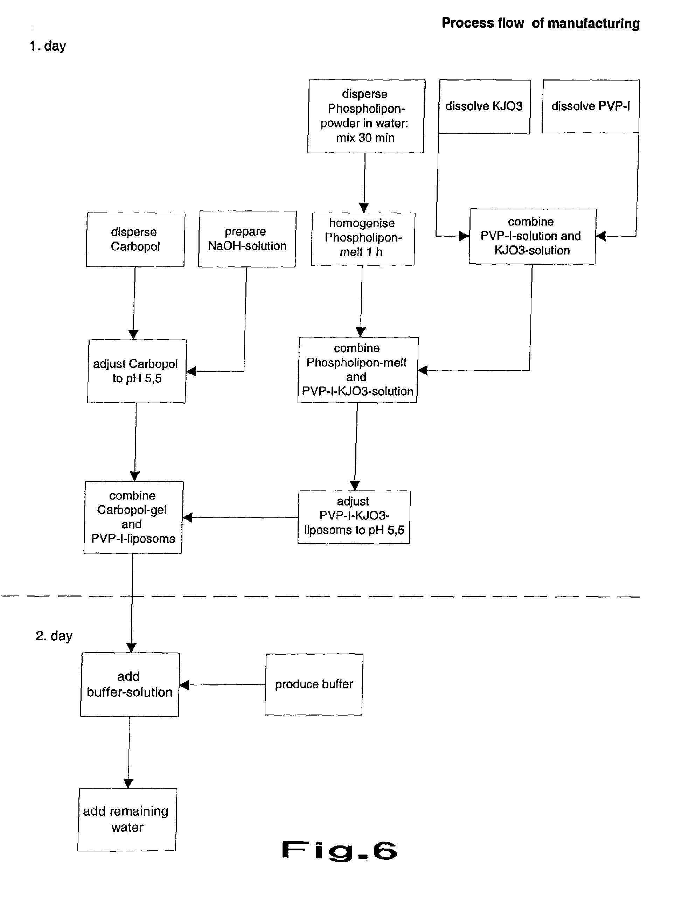

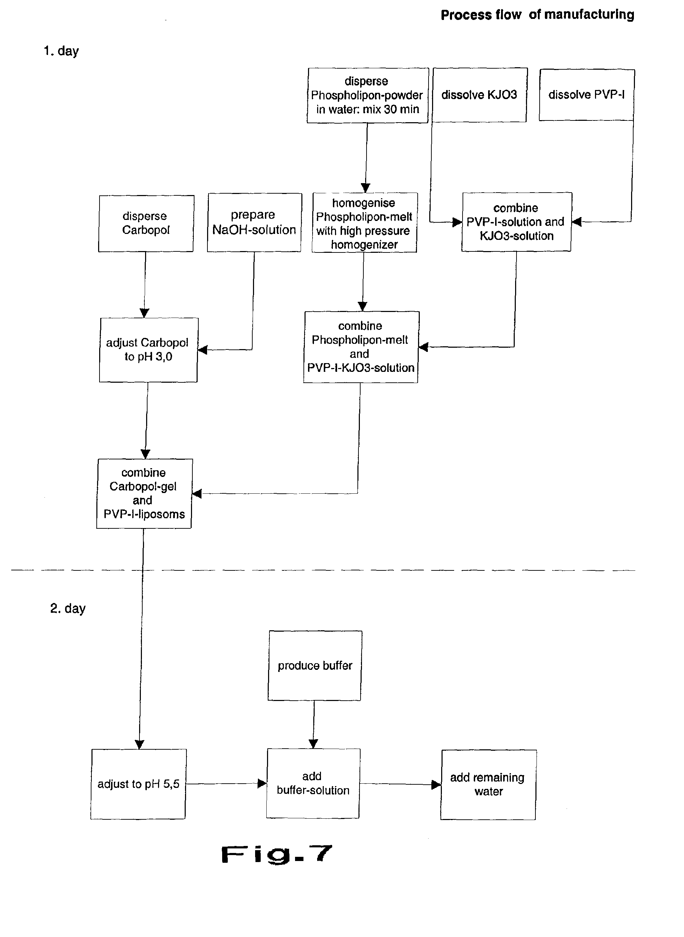

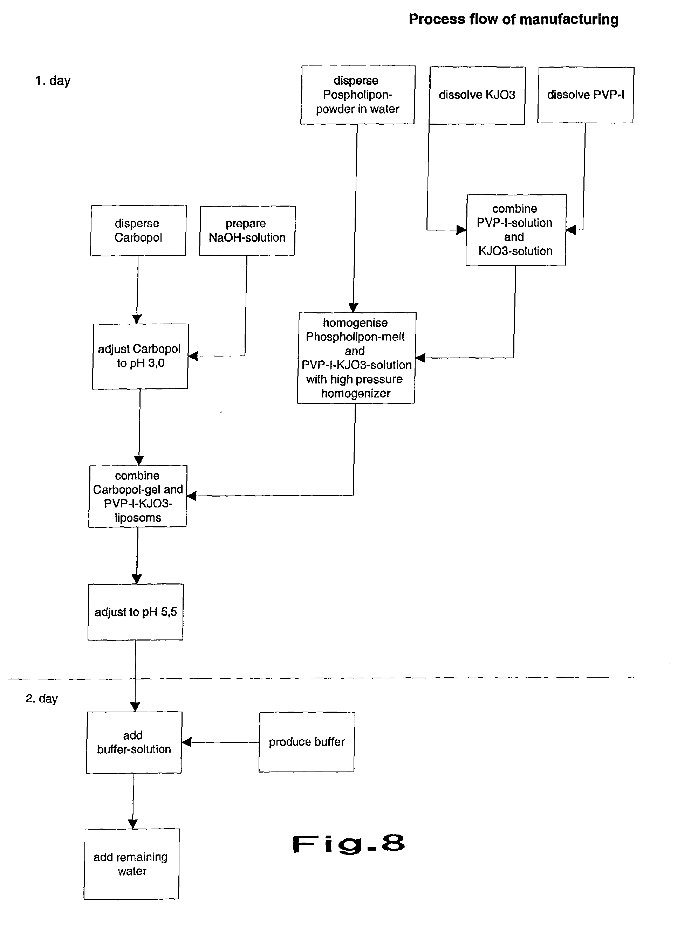

As already mentioned above, a particularly preferred embodiment of the invention is a hydrogel formulation comprising liposomes and PVP-iodine. Such hydrogel formulations usually comprise Phospholipon® 90 H (Aventis, Germany) as liposome-forming material and Carbopol® 980 NF (Noveon Inc, USA) as gel-forming substance. Phospholipon® 90 H is a 90% hydrogenated phosphatidylcholin preparation which is more storage-stable than unhydrogenated phosphatidylcholin preparations. Carbopol® is the trade name for Acrylic acid polymers that are commonly used for formation of pharmaceutically acceptable gels. The preferred hydrogel formulations may also comprise KIO₃, which serves for refurnishing iodine in the preparation. Citric acid and Na₂HPO₄, which are used as buffering agents, advantageously influence the stability of the preparations. Flow charts of the production process are shown in Figures 1 to 8, with Figure 1 illustrating the currently preferred production process. A detailed discussion of this process is given in Example 6.

Examples are set out below that specifically illustrate the production of preferred embodiments of the invention. They are intended to illustrate how preparations according to the invention may be produced and they should by no means be read as limiting the invention to those examples.

### Embodiment Example I - preparation for in vitro tests

In a 1000 ml glass flask, provided with glass beads for increased surface, 51.9 mg cholesterol and 213 mg hydrogenated soy bean lecithin were dissolved in a sufficient amount of a mixture of methanol and chloroform in a 2:1 ratio. The solvent was then evaporated under vacuum until a film was formed on the inner surface of the flask and on the glass beads.

2.4 g PVP iodine (containing about 10 % available iodine) were separately dissolved in 12 ml water.

Again in a separate vessel, 8.77 g sodium: chloride and 1.78 g Na₂HPO₄·2H₂O were dissolved in 400 ml water. Further water was added up to a total volume of 980 ml, and then, approximately 12 ml 1N hydrochloric acid were added to adjust pH to 7.4. This solution was then topped up with water to exactly 1000 ml.

In a fourth vessel, 900 mg saccharose and 57 mg disodium succinate were dissolved in 12 ml water.

The PVP iodine solution was then added to the lipid film in the flask and the mixture was shaken until the film dissolved. The resulting liposome formulation was separated from the hydrated lipids in the flask. The product was centrifuged and the supernatant liquid was discarded. The saccharose solution was added ad 12 ml and the product was again centrifuged. Afterwards the supernatant liquid was again discarded. At this stage, a further washing step, using the saccharose solution or the sodium chloride buffer solution could be carried out.

After the last centrifugation step and discarding of the supernatant, 12 ml sodium chloride buffer solution was added, and the liposomes were homogenously distributed therein. The product was then distributed into vials each containing 2 ml liposome dispersion, and the vials were then subjected to a freeze-drying step.

After the freeze-drying, each vial comprised about 40 mg solids.

The method of Embodiment Example I has a minor disadvantage in that the PVP iodine solution used, due to the high percentage of solids, is rather viscous and thus more difficult to handle.

### Embodiment Example II

In a 2000 ml flask provided with glass beads to increase surface, 173 mg hydrogenated soy bean lecithin and 90 mg disodium succinate were dissolved in approximately 60 ml of a methanol/chloroform mix in a 2:1 ratio. The solvent was removed under vacuum until a film was formed.

4 g PVP iodine (10 % available iodine) were dissolved in 40 ml of the sodium chloride buffer solution described in Embodiment Example I, and were added to the lipid film in the flask. The flask was then shaken until the film dissolved and liposomes were formed.

The product was centrifuged and the supernatant liquid was discarded.

To the thus produced liposome pellet, further 40 ml sodium chloride buffer solution was added, and the centrifuging step was repeated. The supernatant was again discarded. At this stage, the washing step could be repeated where necessary.

After the final centrifuging and decanting step, 40 ml sodium chloride buffer solution was again added to the precipitated liposomes. The homogenous dispersion was then distributed into vials, each vial containing about 2 ml liposome dispersion, and the vials were then subjected to a freeze-drying step. This produced approximately 200 mg freeze-dried solids per vial.

From the freeze-dried solids of Examples I and II, further preparations were made as described in subsequent Embodiment Examples and Test Reports.

Like that of Embodiment Example I, the above-described method uses a hydrating step after film formation in the presence of organic solvents and aims at inclusion rates of 5 to 15 %. These methods generally produce rather large and often multi-lamellar liposomes.

The above-described methods can be modified by a high pressure filtering step through a suitable membrane such as a polycarbonate membrane after the raw liposomes have been formed or after any of the subsequent washing steps or directly by using high pressure homogenisation. This produces much smaller, unilamellar liposomes at increased amounts of encapsulated agent.

Instead of high pressure homogenisation, other prior art methods known to provide small uniform sized liposomes can be employed.

### Embodiment Example III

A hydrophilic (O/W) cream was prepared from 10 g hydrogenated soy bean lecithine/PVP iodine liposomes as described in Embodiment Example II; these were mixed with 4 g Polysorbate 40 (TM), 8 g cetylstearyl alcohol, 8 g glycerol, 24 g white vaseline, and water ad 100 g.

### Embodiment Example IV

An amphiphilic cream was prepared from 10 g hydrogenated soy bean lecithine/povidone iodine liposomes as described in Embodiment Example II; 7.5 g medium chain length tryglyceride, 7 g polyoacneethyleneglycerol monostearate, 6 g cetylstearyl alcohol, 8 g propylene glycol, 25 g white vaseline, and water ad 100 g.

### Embodiment Example V

A hydrophilic ointment which can be rinsed off with water was prepared using 10 g of liposomal PVP iodine as described in Embodiment Example II, 55 g Macrogol 400 (TM), 25 g Macrogol 4000 (TM), and water ad 100 g.

### Embodiment Example VI

A hydrogel was prepared from 4 g liposomal PVP iodine as described in Embodiment Example II, 0.5 g Carbopol® 980 NF (TM), sodium hydroxide ad pH 7.0, water ad 100 g. Further modifications of the above-described embodiments are envisaged.

Thus, the creams of Embodiment Examples IV and V can have an additional content of an agent known to promote the healing of wounds, such as allantoin.

Such an agent will be added in a pharmaceutically useful concentration, in the case of allantoin in the range of 0.1 to 0.5 g, per 100 g of cream. The wound healing agent can be incorporated in the cream base, in which case it will largely be outside the liposomes. It can, however, be partly or mostly incorporated in the liposomes, in which case it will be added at a corresponding suitable stage of the liposome preparation method.

Similar alternatives are easily envisaged on the basis of the further Embodiment

### Examples.

It is also possible to prepare embodiments similar to the above described ones, which comprise an agent capable of promoting the healing of wounds instead of, and not in addition to, the antiseptic agent as e. g. povidone iodine disclosed in the above Embodiment Examples. Presently, it is however preferred to use a wound healing promoting agent (if at all) in addition to an antiseptic agent.

For application of the inventive preparations to a patient, known systems can be used, such as pneumatic pump applicators, two-chamber gas pressure packs, aerosol spray dispensers etc.

In a pneumatic pump applicator, a bellows device is provided between an upstream and a downstream valve, both valves operating one way in the same direction. A supply of pharmaceutical preparation, such as an ointment or gel, is contained in a reservoir upstream of the valves-and-bellows device.

When compressing the bellows, the downstream valve opens and permits a dosed amount of preparation to leave the device for application. When the bellows is extended, this valve shuts and prevents reentry of the preparation. At the same time, the upstream valve opens and permits preparation from the reservoir to enter into the bellows, for release through the downstream valve upon the next compression step of the bellows.

The reservoir is sealed by a closure element which can move through the reservoir like a piston moves in a cylinder. By the stepwise emptying of the reservoir, this closure element is sucked into the reservoir, so that the remaining amount of pharmaceutical preparation in the reservoir is always sealed off, while at the same time the reservoir can be emptied. Such a device is useful for pasty preparations, creams, ointments etc.

In a two-chamber gas pressure pack, the pharmaceutical preparation is contained in a bag of flexible plastics film material. Often, this is high pressure polyethylene.

The bag is contained inside a gas tight pressure vessel which further contains a supply of pressurizing gas, very often a compressed inert gas like nitrogen or air.

The plastic film bag has only one outlet, which is gas-tightly connected to the interior wall of the pressure vessel, surrounding a single opening thereof. The pressurized gas in the vessel tends to compress the bag, driving the pharmaceutical preparation inside the bag out through the opening of the bag and thus through the opening of the vessel. A valve and, in case, sprayhead device is provided in the vessel mouth. Operating the valve releases a spray mist, a jet of liquid or a portion of flowable solid such as cream. Using such a system, solutions, emulsions, creams, oitments and gels can be dosed and applied.

### Embodiment example VII

A Hydrogel was formulated according to the flow chart shown in figure 1. The amounts shown in Table 1 were used either for analytical or scale up preparations.

**Table I**

| Pos. | Substance | Amount (g/100g) | Scale up (kg/1500kg) |
|---|---|---|---|
| A | H₂O | 15,0 | 200,0 |
| A | Phospolipon 90 H | 3,0 | 45,0 |
| B | H₂O | 40,0 | 600,0 |
| B | Carbopol® 980 NF | 1,5 | 22,5 |
| C | H₂O | 2,0 | 30,0 |
| C | KIO₃ | 0,0708 | 1,09 |
| D | H₂O | 20,0 | 300,0 |
| D | PVP-iodine 30/06 Avalaible iodine (10%) | 3,0 | 45,0 |
| E | H₂O | 2,5 | 50,0 |
| F | H₂O | 2,5 | 50,0 |
| G | H₂O | 4,6 | 69,0 |
| G | NaOH solid | 0,46 | 6,9 |
| I | Citric acid, H₂O free | 0,1065 | 1,059 |
| I | Na₂(HPO)₄, H₂O free | 0,225 | 3,37 |
| I | H₂O | 3,0 | 45,0 |
| H | H₂O | ad 100,0 | ad 1500 |

Pos. stands for Position (see also below Table 2). Phospholipon® 90 H was purchased from Aventis (Germany). Carbopol® 980 NF was purchased from (Noveon Inc., USA) and PVP Iodine 30/06 was purchased from BASF (Germany).

In Table 2, column 2 the exact order of steps and the parameters of each step are given (see also Figure 1). Column 3 discusses non-exclusive alternatives. All steps were performed at room temperature except where indicated otherwise. All substances were of a purity grade common for pharmaceutical preparations such as described in the British Pharmacopeia.

**Table II**

| No. | Embodiment example VII | Alternatives |
|---|---|---|
| 1 | Carbopol 980 NF is mixed into H₂O without agglomeration (Pos. B). Stirring for 30 min at approx. 30 upm (units per minute) in conventional stirrer. Visual control for Polyacrylic acid-agglomerates. If necessary, homogenize gel in conventional homogenisator for 2 min at 3000 upm. Subsequently stir gel for 30 min at 30 upm in conventional stirrer. Eventually control again for Polayacrylicacid-agglomerates. If present, remove them and stir again for 15 min at 30 upm. Eventually homogenize again. Let gel swell for at least 14 h. | Substances: Other gel-forming substances may be used. Homogenization time can vary: shorten to 1 min prolong to 10 min (caution! gel structure may be destroyed) Stirring time can be altered as desired. Only condition is that gel is free of agglomerates at the end. Swelling time may be altered from 15 min to 5 days. Preferably the gel has formed before other substances are added. Adjustment of pH to 2-8 may be performed at this stage. Adjustment to pH 3-6 is preferred. |
| 2 | Dissolve H₂O and KIO₃ completely in a suitable vessel (Pos. C). Alternatively a 30-40% KIO₃ solution may be used. | H₂O-temperature may be adjusted to anywhere between ambient temperature and 100 °C. KIO₃ is not obligatory. |
| 3 | Dissolve NaOH completely in H₂O (Pos. G). | NaOH is used in concentrations common for pharmaceutical preparations. Other Bases or substances suggested by the supplier of the gel forming substances may also be used for formation of gel structure as e.g. KOH, Triethanolamine, 2-Amino-2-methyl-1-propanol, tris(hydroacnemethyl)aminoethan, 2-hydroacnepropyl-ethylen-diamine, diisopropanolamine. |
| 4 | Mix PVP-iodine into H₂O while stirring at 1000 upm in conventional stirrer (Pos. D). Stir mixture for futher 60-70 min at 1000 upm until it is completely dissolved. | Stirring time and speed can be altered arbitrarily. Important: PVP-Iodine has to be dissolved completely. |
| 5 | Warm H₂O to 65 °C while stirring with 1000 upm in conventional stirrer. Then add slowly | Possible temperature range: 40 °C - 120 °C. 50 °C -75 °C is preferred because of phase transition |
| | Phospholipon® 90 H (Pos. A). Take care that no agglomerates are formed. Stir dispersion for further 90 min at 65°C - 70 °C and 1000 upm. Subsequently cool liposomal dispersion to ≤30 °C while stirring at 500 upm | temperature. Other liposome-forming materials or mixtures thereof may be used. Stirring time and speed: Is dependent on equipment. A complete dispersion has to be achieved. Apparatus of the rotor/stator principle, high pressure homogenisators, ultrasound or extrusion technology may also be used for stirring. |
| 6 | By adding the NaOH-solution (No. 3) the gel is adjusted to a pH of 3.0 (± 0,2). | Further processing to a gel may be feasible without pH pre-adjustment and is dependent on the gel-forming substance |
| 7 | The KIO₃ solution (No.2) is added to the PVP-Iodine solution (No. 4) while stirring at 1000 upm. Stirring continued for at least 60 min. | Reaction between KIO₃ and PVP-iodine is time dependent. To ensure a complete reaction, the stirring time has to adapted accordingly. Thus, stirring time may be between 10 min and 2 h |
| 8 | The PVP-iodine- KIO₃ -solution is pumped into the liposomal dispersion (No. 5). Subsequently it is stirred for 30 min at 1000 upm: | Stirring time is variable depending on until when an homogeneous mixture has formed. |
| 9 | The PVP-iodine- KIO₃-liposomes-dispesion is added to the gel (No. 6). It is stirred for 30 min at 30 upm. Subsequently homogenization is performed by forced circulation pumping for 2 min at 2800 upm. After checking for agglomerates, it may be homogenized for further 1 - 2 min. | Stirring time is variable depending on until when an homogeneous mixture has formed. Stirring time should be as short as possible so that gel structure gets not disrupted. |
| 10 | Remove agglomerates if present. Add 50,0 kg NaOH-solution (in the scale up, point 3) while stirring at 30 upm. Stir for further 30 min at 30 upm at ≤30 °C. Cool if necessary. Determine pH and add additional NaOH until an pH of 5.5 (±0.2) is achieved. After each adding step stir for 20 min. After each adding step homogenize by circulation pressure pumping for 15 sec at 1000 upm. After adjustment of pH stir for further 15 min at 30 upm. Check pH and correct if necessary. | Adjust stirring time and speed to gel quality. Amounts of NaOH may vary. Adding of base by step wise adjustment until desired pH is achieved. |
| | After successful pH adjustment add remaining H₂O amount which depends on the amount of NaOH used. | |
| 11 | Mix buffer solution at 30 °C while stirring until it is completely dissolved (Pos. I). | Temperature can be raised to 40 °C. Other suitable buffers may also be used. |
| 12 | Buffer solution is added to the product (No. 10) while stirring for 15 min at 30 upm. Degas by application of vacuum. | The desired product quality (storage stability) is achieved by addition of the buffer. Stirring time is variable depending on until when an homogeneous mixture has formed. Degasing may be achieved by other means than vacuum. |
| 13 | Add the remaining H₂O-amount (Pos. H) and stir for 30 min at 25 upm Optionally homogenization may be performed by circulation pressure pumping for 15 sec at 1000 upm. Stir for another 30 min. Check visually for agglomerates | Stirring time is variable depending on until when an homogeneous mixture has formed. |

Positions E and F of Table I are used for washing the KIO₃- and the PVP-iodine vessels (points 2 and 4 of Table II).

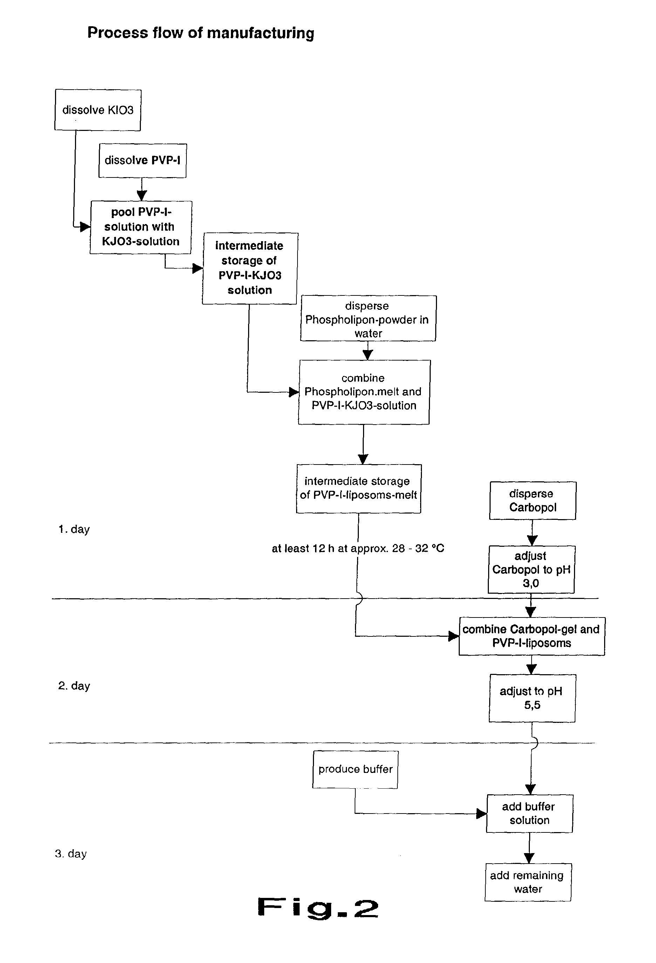

As mentioned above, the Hydrogel-Formulation is produced according to the method set out in Table 2 and Figure 1. Alternative methods become obvious from the flow charts of figures 2 to 8. The individual steps may be performed as set out above.

Using inventive preparations efficiency tests were then carried out, as follows:

### Test I

This was an in-vitro-test of the bactericidal effect provided by an inventive povidone iodine liposome preparation. The test was based on the quantitative suspension test as described in "Richtlinien der Deutschen Gesellschaft für Hygiene und Mikrobiologie", 1989. In this test, the bactericidal agent is used to kill staphylococcus aureus (ATCC 29213), a major problem in hospital hygiene.

The liposome preparation used was that of Embodiment Example I. At different contact times between 1 und 120 minutes, the minimum concentration of the preparation in water was determined which was capable of killing the staphilococci.

The results are shown in Table 3.

**TABLE III**

| Contact Time (Minutes) | Bactericidal Concentration |
|---|---|
| 1, 2, 3, 4 | ≥ 0.060 % |
| 5, 30, 60 | ≥ 0.015 % |
| 120 | ≥ 0.007 % |

The results show that at short contact times (between 1 and 4 minutes) the bactericidal concentration is as low as 0.06 % and that at long contact times (120 minutes) the bactericidal concentration can be as low as 0.007 %.

### Test II

The virucidal and chlamydicidal activity of liposomal PVP-iodine has been studied, in cell cultures, by Wutzler et al., 9th European Congress for Clinical Microbiology and Infection Diseases, Berlin, March 1999. In cell cultures, liposomal PVP-iodine is highly effective against herpes simplex virus type 1 and adenovirus tpye 8, while the long-term cytotoxicity experiments indicated that the liposomal form is better tolerated than aqueous PVP-iodine by the majority of cell lines tested. PVP-iodine in liposomal form is not genotoxic.

### Test III

A 3% PVP-iodine hydrogel liposomal preparation was compared with a 3% PVP-iodine ointment, where the active agent was not in liposomal form. The agent was applied to standardized in vitro cultures of rat skin and peritoneal explants, as a screening for tissue compatibility of skin and wound antiinfectives.

The growth rate of the cultured explants was studied after 30 minutes exposure and incubation with a test substance.

Again, the substantially better toleration of the liposomal preparation was clearly shown in the results, in terms of peritoneum growth rate and skin growth rate.

With the ointment, the peritoneum growth rate reached 85%, and the skin growth rate reached 90%; with the liposomal hydrogel formulation, the peritoneum growth rate was 96%, and the skin growth rate was 108%; these values are to be compared with 100% values in a control test using Ringer's solution as the agent.

### Test IV

The toleration of liposomal PVP-iodine solutions for nasal applications was studied by investigating the influence of different test substances on ciliated epithelium cells, the most sensible cells of the mucous membrane. A cytotoxic damage of these cells which would cause a restriction of the mucociliar clearance can be determined by a detectable decrease of the ciliary vibration.

Human ciliated epithelium cells were analysed by an in-vitro method which enables the determination of the ciliary activity or ciliary vibration. The corresponding cells were exposed and incubated with 100 je. 1 test substance at a temperature of 37 C. After an incubation period of 5 minutes the ciliary vibration was measured.

By using this in-vitro method a nutriant solution (Dulbeco) as standard, a 0.2% chlorohexidine solution (typical antiseptic agent), conventional polyvidone iodine solutions (Betaisodona) of different concentrations (5.0%, 2.5% and 1.25% PVP-iodine) and a liposomal solution containing 4.5% of PVP-iodine were tested.

The substantially better toleration of the liposomal preparation was clearly shown in the results: if the ciliated epithelium cells were exposed to the Betaisodona solutions containing 5.0% or 2.5% PVP-iodine, no ciliary activity could be observed after the incubation period. Treating the cells with a chlorohexidine solution led to a decrease of the measured ciliary vibration in comparison to the standard (nutriant solution). The low concentrated Betaisodona solution containing 1.25% PVP-iodine, didn't cause a detectable decrease of the ciliary activity. With respect to the measured ciliary vibration no differences to the standard (nutrian solution) could be determined by exposing the human ciliated epithelium cells to the concentrated liposomal 4.5% PVP-iodine solution.

These results indicate that the liposomal formulation is well tolerated for nasal application and advantageous with respect to for e. g. chlorohexidine or conventional Betaisodona solutions.

## Claims

1. Method for producing a pharmaceutical preparation for the treatment of acne, **characterized in, that** the preparation comprises at least one antiseptic compound in a pharmaceutically effective amount combined with a particulate, pharmaceutically acceptable carrier.

2. Method according to claim 1,
**characterized in, that** the particular carrier comprises liposomes, microspheres, nanoparticles, "Large Porous Particles", laserpuls-polymer coated molecules particles and/or other micelles.

3. Method according to claim 1 or 2,
**characterized in, that** the antiseptic compound comprises oxygen- and halogen-releasing compounds, preferably iodine and iodine complexes, and/or metal compounds, preferably silver- and mercury-compounds.

4. Method according to claim 3,
**characterized in, that** the antiseptic compound is PVP-iodine.

5. Method according to one of claims 1 to 4,
**characterized in, that** the preparation comprises additional antiseptic compounds such as organic disinfectants including formaldehyde-releasing compounds, phenolic compounds including alkyl- and aryl-phenolic compounds, chinolines and acridines, hexahydropyrimidines, quartenary ammonia compounds, imines and salts thereof and guanidines.

6. Method according to one of claims 1 to 5,
**characterized in, that** the preparation comprises additionally wound-healing promoting agents, that promote granulation and epithel-formation such as dexpanthenol, allantoines, azulenes, tannins, vitamins, preferably vitamin B and derivatives thereof.

7. Method according to one of the preceding claims,
**characterized in, that** the particulate carriers, particularly liposomes, have a size in a range between approximately 1 µm and approximately 100 µm, preferably between approximately 1 µm and approximately 50 µm and most preferably between approximately 1 µm and approximately 25 µm.

8. Method according to one of the preceding claims,
**characterized in, that** the particulate carriers, particularly liposomes, release the active compound(s) over an extended time period, preferably over an time period of several hours duration.

9. Preparation according to claim 8,
**characterized in, that** the particulate carriers, particularly liposomes, release the active compound(s) at approximately the same release rate over the time of the release.

10. Method according to one of the preceding claims,
**characterized in, that** the preparation comprises additives and adjuvants such as conserving agents, antioxidants and consistency-forming additives.

11. Method according to one of the preceding claims,
wherein the preparation is provided in the form of a solution, suspension, dispersion, ointment, spray, lotion, cream, gel or hydrogel comprising the compound-loaded particulate carriers, preferably in the form of a liposomal solution, suspension, dispersion, ointment, lotion, cream, gel or hydrogel.

12. Method according to one of the preceding claims,
wherein the preparation is provided in the form of a pharmaceutical solution-, suspension-, dispersion-, ointment-, lotion-, cream-, gel- or hydrogel-formulation that comprises:
• liposomes comprising a pharmaceutically acceptable liposomal membrane forming substance and
• a 0,1% to 5% PVP-iodine solution (with approximately 10% available iodine in the PVP-complex,
wherein the liposomes are of a size with diameters between approximately 0,01 µm and approximately 50 µm and, in case, the formulation additionally comprises customary additives, adjuvants and auxiliary substances of a pharmaceutical solution-, suspension-, dispersion-, ointment-, lotion-, cream-, gel- or hydrogel-formulation.

13. Method according to claim 12,
**characterized in, that** the liposomes are of a size with diameters between approximately 1 µm and approximately 25 µm.

14. Method according to one of the preceding claims, wherein the preparation is suitable for the treatment of acne, particularly *of Acne vulgaris, Acne aestivalis, Acne cosmetica, Acne excoriée des jeunes filles, Acne fulminans, Acne neonatorum, Acne venenata* and *Acne tetrade.*

15. Method according to claim 14,
wherein the preparation is suitable for the treatment of various forms *of Acne vulgaris* such as *Acne comedonica, Acne papulopustulosa* and *Acne conglobata.*

16. Method according to one of the preceding claims,
wherein the preparation can be used for the topic treatment of the various acne forms preferably in the face, on the breast, on the back and the extremities.

17. Method according to one of the preceding claims,
wherein the preparation can be used for the topic treatment of the bacterial and viral inflammations and infections as well the hyperkeratosis and/or the hyperseborrhea that occur with the different acne forms.
